# EUROPEAN PATENT APPLICATION

(11) **EP 2 628 750 A2**
(43) Date of publication of application: **21.08.2013**
(21) Application number: 13160710.3
(22) Date of filing: 28.03.2008
(51) Int. Cl.: C07K 14/71, A61K 39/00, G01N 33/50, G01N 33/564, G01N 33/574, G01N 33/68, A61K 39/395, C07K 16/32

(54) **Targeted identification of immunogenic peptides**

(30) Priority: 10.03.2008 US 45402; 10.03.2008 CA 2622036
(62) Divisional of application: 08719072.4
(71) Applicant: The Henry M. Jackson Foundation for the Advancement of Military Medicine, Inc., Rockville, MD 20852 (US)
(72) Inventor: Peoples, George E., San Antonio, TX 78209 (US); Storrer, Catherine E., Columbia, MD 21044 (US); Ponniah, Sathibalan, Columbia, MD 21044 (US); Flora, Michael, Mt Airy, MD 21771 (US)
(74) Representative: Steinecke, Peter

(57) **Abstract**

This invention relates generally to identifying peptide sequences involved in antibody binding to any protein for synthesis of vaccine treatments. This method allows for a more manageable vaccine peptide discovery and specific generation of unique immunogenic peptides from self-tumor associated proteins and/or foreign proteins from infectious organisms for specific and/or enhanced expression only in the presence of the antibody.

## Description

### Rights in the Invention

This invention was made, in part, with support for the United States government and the United States government may have an interest in this invention.

### Background of the Invention

### 1. Field of the Invention

This invention, in the field of immunology/immunotherapy, vaccine discovery and development, relates generally to the identification of immunogenic peptides from regions of proteins and molecules that are involved in the binding interactions with polyclonal and monoclonal antibodies and other specific binding peptides/molecules. The present invention is directed to methods for identification and use of the peptides for preventing, suppressing and treating immune-related diseases. Specifically, the invention provides therapy that result in clinical improvement in cancer patients.

### 2. Description of the Background

Autoimmune diseases are characterized by an unwanted and unwarranted attack by the immune system on the tissues of the host. While the mechanism for progress of these diseases is not well understood, at least some of the details with respect to antigen presentation are known. It is thought that antigens, including autoantigens, are processed by antigen-presenting cells (APC), and the resulting fragments are then associated with one of the cell surface proteins encoded by the major histocompatibility complex (MHC). As a result, recognition of a peptide antigen is said to be MHC "restricted." When the MHC/antigen fragment complex binds to a complementary T cell receptor (TCR) on the surface of a T lymphocyte, activation and proliferation of the clone or subpopulation of T cells result bearing that particular TCR. Once activated, T cells have the capacity to regulate other cells of the immune system which display the processed antigen and to destroy the cells or tissues which carry epitopes of the recognized antigen.

Antibody therapies in which antibodies are directed to MHC molecules and CD4 molecules have been generally successful in several animal models of autoimmunity. However, these approaches may be too nonspecific and potentially overly suppressive. This may be because 70% of T cells bear the CD4 marker and because all T cell-mediated responses and most antibody responses require MHC-associated antigen presentation.

A major difficulty with present approaches is that they require the use of complex biological preparations which do not comprise well-defined therapeutic agents. Such preparations suffer from complex production and maintenance requirements (e.g., the need for sterility and large quantities of medium for producing large number of "vaccine" T cells), and lack reproducibility from batch to batch. To be useful in humans, T cell "vaccine" preparations must be both autologous and individually specific. This means they must be uniquely tailored for each patient. Furthermore, the presence of additional antigens on the surface of such T cells may result in a broader, possibly detrimental, immune response not limited to the desired T cell clones (Offner et al., J. Neuroimmunol. 21:13-22 (1989).

There is a need, therefore, for agents and pharmaceutical compositions which have the properties of specificity for the targeted immune response. These agents and compositions should also have predictability in their selection, convenience and reproducibility of preparation, and sufficient definition in order to permit precise control of dosage.

An effective vaccine is capable of generating a long-lasting immunity while being relatively harmless to the recipient. Attenuated organisms and purified antigens from organisms have traditionally been used as vaccines. However, such agents often produce deleterious side effects or fail to protect against subsequent challenges. Because of the inherent difficulties in growing pathogenic organisms and producing effective vaccines from them, many viral, bacterial and parasitic diseases have no effective vaccine.

A further difficulty with the use of peptides as vaccines is that, in most instances, peptides alone are not good immunogens. It is a well known phenomenon that most immune responses to peptide antigens are T cell-dependent. Accordingly, "carrier" molecules have been attached to peptide antigens that bind, for example, to B cell surface immunoglobulin in order to generate a high affinity, IgG response. In other words, nonresponsiveness to peptide antigens may sometimes be overcome by attaching another peptide that induces helper T cell activity.

In general, peptides that induce helper T cell activity are generated by B cells from enzymatic digestion of native proteins internalized by way of an antibody receptor. These T cell stimulating peptides are then presented on the surface of the B cell in association with class II major histocompatibility complex (MHC) molecules. In a similar fashion, peptides that induce cytotoxic T cell activity may be generated by accessory cells, including B cells. These peptides are presented on the cell surface of accessory cells in association with class I MHC molecules. As used herein, the term "T cell stimulatory peptide" means any peptide which activates or stimulates T cells, including (but not limited to) helper T cells and/or cytotoxic T cells.

Peptides represent a promising approach to the production and design of vaccines. However, the difficulties in making peptides that induce the desired immune response have hampered their success. This includes the difficulties inherent in making peptides that closely mimic the native structure of antigenic determinants.

These antigenic determinants, or epitopes, of a protein antigen represent the sites that are recognized as binding sites by certain immune components such as antibodies or immunocompetent cells. While epitopes are defined only in a functional sense, i.e. by their ability to bind to antibodies or immunocompetent cells, there is a structural basis for their immunological activity.

Epitopes are classified as either being continuous (e.g. linear) and discontinuous (non-linear). Discontinuous epitopes are composed of sequences of amino acids throughout an antigen and rely on the tertiary structure or folding of the protein to bring the sequences together and form the epitope. In contrast, continuous epitopes are linear peptide fragments of the antigen that are able to bind to antibodies raised against the intact antigen.

Many antigens have been studied as possible serum markers for different types of cancer because the serum concentration of the specific antigen may be an indication of the cancer stage in an untreated person. As such, it would be advantageous to develop immunological reagents that react with the antigen. More specifically, it would be advantageous to develop immunological reagents that react with the epitopes of the protein antigen.

Conventional methods using biochemical and biophysical properties have attempted to determine the location of probable peptide epitopes. These methods include a careful screening of a protein's primary structure, searching for critical turns, helices, and even the folding of the protein in the tertiary structure. Continuous epitopes are structurally less complicated and therefore may be easier to locate. However, the ability to predict the location, length and potency of the site is limited.

Various other methods have been used to identify and predict the location of continuous epitopes in proteins by analyzing certain features of their primary structure. For example, parameters such as hydrophilicity, accessibility and mobility of short segments of polypeptide chains have been correlated with the location of epitopes.

Hydrophilicity has been used as the basis for determining protein epitopes by analyzing an amino acid sequence in order to find the point of greatest local hydrophilicity. As discussed in U.S. Pat. No. 4,554,101, each amino acid is assigned a relative hydrophilicity numerical value which is then averaged according to local hydrophilicity so that the locations of the highest local average hydrophilicity values represent the locations of the continuous epitopes. However, this method does not provide any information as to the optimal length of the continuous epitope. Similarly, U.S. Pat. No. 6,780,598 B1 determines the immunopotency of an epitope by providing a ranking system delineating between dominant and subdominant epitopes.

Computer-driven algorithms have been devised to take advantage of the biochemical properties of amino acids in a protein sequence by sorting information to search for T cell epitopes. These algorithms have been used to search the amino acid sequence of a given protein for characteristics known to be common to immunogenic peptides. They can often locate regions that are likely to induce cellular immune response *in vitro.* Computer-driven algorithms can identify regions of proteins that contain epitopes which are less variable among geographic isolates, or regions of each geographic isolate's more variable proteins, or perform as a preliminary tool to evaluate the evolution of immune response to an individual's own quasi species.

Peptides presented in conjunction with class I MHC molecules are derived from foreign or self protein antigens that have been synthesized in the cytoplasm. Peptides presented with class II MHC molecules are usually derived from exogenous protein antigens. Peptides binding to class I molecules are usually shorter (about 8-10 amino acid residues) than those that bind to class II molecules (8 to greater than 20 residues).

Identification of T cell epitopes within protein antigens has traditionally been accomplished using a variety of methods. These include the use of whole and fragmented native or recombinant antigenic protein, as well as the more commonly employed "overlapping peptide" method for the identification of T cell epitopes within protein antigens which involves the synthesis of overlapping peptides spanning the entire sequence of a given protein. Peptides are then tested for their capacity to stimulate T cell cytotoxic or proliferation responses *in vitro.*

The overlapping peptide method is both cost and labor intensive. For example, to perform an assay using 15 amino acid long peptides overlapping by 5 amino acids spanning a given antigen of length n (a small subset of the possible 15-mers spanning the protein), one would need to construct and assay (n/5)-1 peptides. For most types of analyses, this number would be prohibitive.

Accordingly, a simple method to identify immunogenic peptides from regions of self-proteins and other proteins and molecules involved in binding interactions with polyclonal and monoclonal antibodies is needed.

### Description of the Figures

**Figure 1** depicts one embodiment of the invention in which a unique immunogenic region of the HER-2/neu is identified.
**Figure 2** shows the HER2/neu sequence (SEQ ID NO: 1) and highlights the antibody-binding site of Herceptin to the HER2/new protein. Also shown is the E75 vaccine sequence (SEQ ID NO: 2) and the HER2 loops binding to Herceptin (SEQ ID NO: 3).

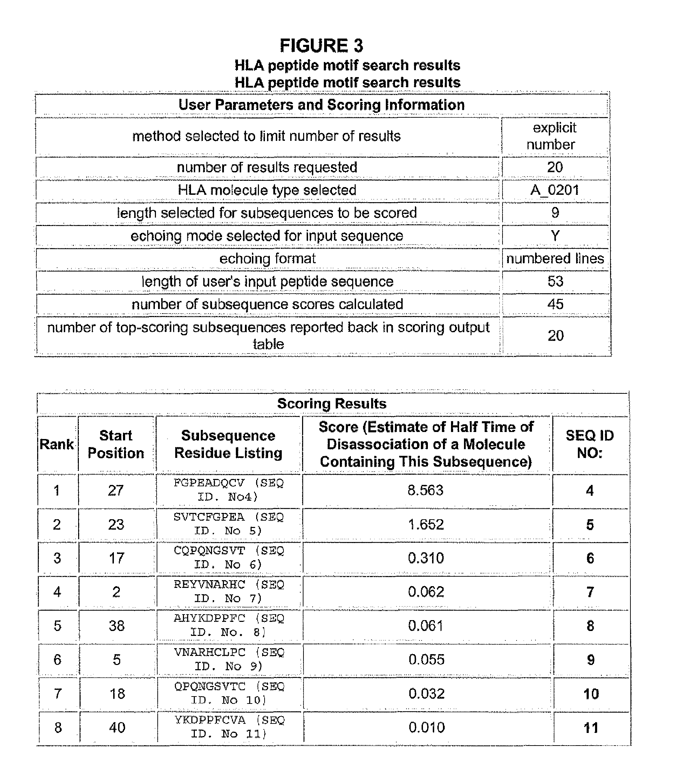

**Figure 3** shows HLA peptide motif search results and scoring results for SEQ ID NOs: 4-11.

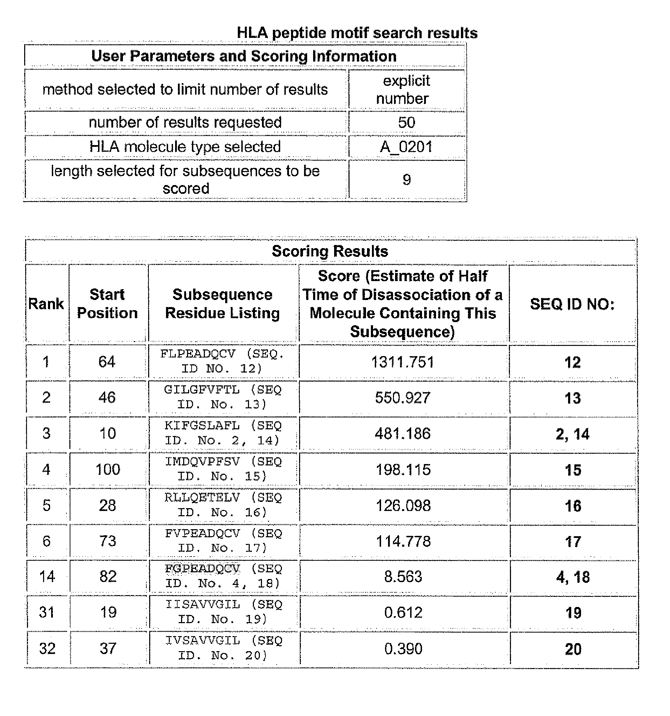

**Figure 4** shows amino acid substitutions for increased binding affinity of native peptide vaccine development. A table indicating HLA peptide motif search results, as well as the scoring results for SEQ ID NOs: 12-20 are included.

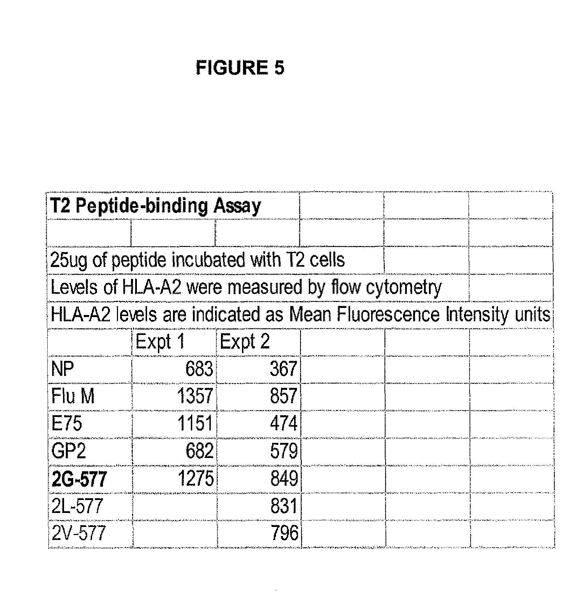

**Figure 5** depicts a T2 Peptide-binding assay, in which 25µg of peptide were incubated with T2 cells, levels of HLA-A2 levels were measured by flow cytometry and are indicated as Mean Fluorescence Intensity units.

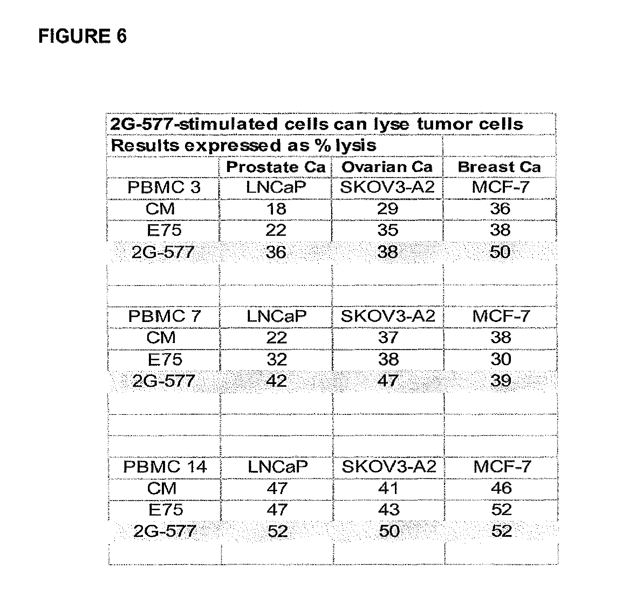

**Figure 6** is a table depicting an experiment looking at whether 2G-577- and E75-stimulated cells can lyse tumor cells, in prostate cancer, ovarian cancer, and breast cancer, with results expressed as % lysis.
**Figure 7** is a table showing enhanced lysis of Herceptin-treated tumor targets by E75, GP2, 2G-577, 2V-577, and 2L-577-stimulated cells, with results expressed as % lysis. These data suggest the benefit of combination therapy with Herceptin treatment and vaccine-induced T cell targeting for synergistic tumor cell killing.
**Figure 8** is a table showing enhanced lysis of Herceptin-treated tumor targets by 2G-577-stimulated cells from breast cancer patients, with results expressed as % lysis.

### Summary of the Invention

The invention overcomes the problems and disadvantages associated with current methods and provides tools and methods of generating an immune response in a patient in need thereof.

One embodiment of the invention is directed to a method for synthesizing an immunogenic peptide from a self-protein comprising the steps of identifying one or more peptide sequences of a self protein that are directly or indirectly involved with antibody-binding, subjecting the one or more peptide sequences to an algorithm that identifies sequences suspected of being immunogenic, screening all peptide fragments from the one or more peptide sequences, and identifying an immunogenic peptide of the protein fragment wherein the antibody-binding interaction is polyclonal or monoclonal. Further, a patient is treated with the immunogenic peptide to generate an immune response.

Another embodiment of the invention is directed to immunogenic peptides identified by the method described above.

Another embodiment of the invention is directed to an immunogenic peptide that produces an immune response to a self-protein.

Another embodiment is directed to a method of presenting epitopes for recognition by the immune system to generate an immune response comprising the steps of identifying a protein fragment that is recognized by a pool of unused and immunoreactive T cells, subjecting the protein fragment to an algorithm, identifying one or more specific sequences of the protein fragment that is immunogenic, synthesizing at least one immunogenic peptide corresponding to the sequence and treating a patient with the immunogenic peptide to generate an immune response. Further, an antibody that binds to the protein is generated.

Another embodiment of the invention is directed to immunogenic peptides identified by the method described above.

Another embodiment of the invention is directed to a vaccine comprising the immunogenic peptides described above.

Another embodiment of the invention is directed to a vaccine comprising antibodies that react with the immunogenic peptides described above.

Another embodiment of the invention is directed to a method of identifying a vaccine treatment comprising the steps of binding an antibody to a protein molecule forming a complex, subjecting the complex to proteasome digestion, obtaining digestion products comprising peptides, and identifying an immunogenic peptide sequence from the digestion products.

Another embodiment of the invention is directed to a method of identifying a patient-specific treatment comprising the steps of obtaining a pre-existing immuno-reactive precursor to said patient's AGIE/ABIE, culturing tumor cells obtained from said patient, incubating the cultured tumor cells that are reactive against generated antibodies, examining dataset responses in presence and absence of the generated antibodies and identifying the patient-specific immunogenic epitopes. This method may include the generation of antibodies that are reactive against self antigens, the generation of antibodies that are reactive against foreign antigens, and/or the generation of antibodies, once administered to said patient, that are therapeutic or prophylactic.

Another embodiment of the invention is directed to a method of identifying a vaccine treatment comprising the steps of binding an antibody to a protein molecule with a specific binding activity forming a complex, subjecting the complex to proteasome digestion, obtaining digestion producing comprising peptides, identifying an immunogenic peptide sequence from the digestion products.

Other embodiments and technical advantages of the invention are set forth below and may be apparent from the drawings and the description of the invention which follow, or may be learned from the practice of the invention.

In one aspect of the present invention there is provided a therapeutically effective composition comprising a peptide that represents a portion of the sequence of HER2/neu antigen, wherein the peptide contains the sequence of SEQ ID NO: 2 (E75 = KIFGSLAFL), and further comprising an antibody that binds to an immunogenic region of the antigen. In one embodiment, the composition is a pharmaceutical composition. In one embodiment, the peptide sequence is substantially within the immunogenic region of the antibody binding site. In one embodiment, the antibody is trastuzumab.

In one aspect of the present invention, there is provided a vaccine which comprises the composition, wherein the vaccine is therapeutically effective in the treatment of cancer. In one embodiment, the cancer is breast cancer.

In one aspect of the present invention, there is provided a pharmaceutical composition for use in the treatment of cancer comprising a peptide that represents a portion of the sequence of HER2/neu antigen, wherein the peptide contains the sequence of SEQ ID NO: 2 (E75 = KIFGSLAFL), and further comprising an antibody that binds to an immunogenic region of the antigen, wherein the antibody is trastuzumab.

In one aspect of the present invention, there is provided a peptide which represents a portion of the sequence of HER2/neu antigen, and contains the sequence of SEQ ID NO: 2 (E75 = KIFGSLAFL) for treating a patient in a method comprising, in any order:
a. administering a therapeutically effective amount of an antibody to the patient, wherein the antibody is trastuzumab; and
b. administering a therapeutically effective amount of the peptide to the patient. In one embodiment, the peptide is for administration before, after or at the same time as administration of the antibody. In one embodiment, the administration of the peptide and administration of the antibody have a synergistic effect on the patient. In one embodiment, the patient is a breast cancer patient.

In one aspect of the present invention, there is provided a therapeutically effective composition comprising a peptide that represents a portion of the sequence of HER2/neu antigen, wherein the peptide contains the sequence of GP2 (IISAVVGIL - SEQ ID NO: 19) or GP2', (IVSAVVGIL, SEQ ID. No 20), and further comprising an antibody that binds to an immunogenic region of the antigen. In one embodiment, the peptide sequence is substantially within the immunogenic region of the antibody binding site. In one embodiment, the antibody is trastuzumab.

In one aspect of the present invention, there is provided a vaccine comprising a composition comprising a peptide that represents a portion of the sequence of HER2/neu antigen, wherein the peptide contains the sequence of SEQ ID NO: 19 (GP2 = IISAVVGIL) or SEQ ID NO: 20 (GP2' = IVSAVVGIL), and further comprising an antibody that binds to an immunogenic region of the antigen, which is therapeutically effective in the treatment of cancer. In one embodiment, vaccine is for treating breast cancer.

In one aspect of the present invention, there is provided a pharmaceutical composition for use in the treatment of cancer comprising a peptide that represents a portion of the sequence of HER2/neu antigen, wherein the peptide contains the sequence of SEQ ID NO: 19 (GP2 = IISAVVGIL) or SEQ ID NO: 20 (GP2' = IVSAVVGIL), and further comprising an antibody that binds to an immunogenic region of the antigen, wherein the antibody is trastuzumab.

In one aspect of the present invention, there is provided a peptide which represents a portion of the sequence of HER2/neu antigen, and which contains the sequence of SEQ ID NO: 19 (GP2 = IISAVVGIL) or SEQ ID NO: 20 (GP2' = IVSAVVGIL) for use in treating a patient in a method comprising in any order:
a. administering a therapeutically effective amount of an antibody to the patient, wherein the antibody is trastuzumab; and
b. administering a therapeutically effective amount of the peptide to the patient. In one embodiment, the peptide is for administration before, after or at the same time as administration of the antibody. In one embodiment, administration of the peptide and administration of the antibody have a synergistic effect on the patient. In one embodiment, the patient is a breast cancer patient.

In one aspect of the present invention, there is provided a peptide which has an amino acid sequence of (1) at least 7 contiguous amino acid residues of a HER2/neu protein e.g. at least 8 amino acid residues; or (2) a variant thereof which comprises at least 7 amino acid residues e.g. at least 8 amino acid residues, for administration in combination with an anti HER2/neu antibody to treat a cancer caused by over-expression of the HER2/neu protein, wherein the administration of the peptide is sequentially, simultaneously or separate to administration of the antibody, wherein optionally the peptide is not the full length HER2/neu protein

In one embodiment, the variant comprises a mutation e.g. a substitution, for improved binding to MHC claim I molecules. In one embodiment, the peptide is a variant as compared to the wild-type sequence of the HER2/neu protein and contains, for example, a substitution of the amino acid residues at position 2 to leucine or methionine and/or substitution to valine at position 9 for improved binding to for example a HLA-A2 molecule.

In one embodiment, the peptide comprises an amino acid substitution at position 2 and/or position 9 of the peptide. In one embodiment, the variant comprises an amino acid substitution which replaces the amino acid residue at position 2 with (1) a glycine residue; (2) leucine residue (3) methionine or (4) valine residue. In one embodiment, the peptide comprises a mutation at amino acid residue at position 9. In one embodiment, the mutation is an amino acid substitution which replaces the amino acid residue at position 9 with a valine residue. In one embodiment, the variant comprises an additional group e.g. a methylene group at position 4.

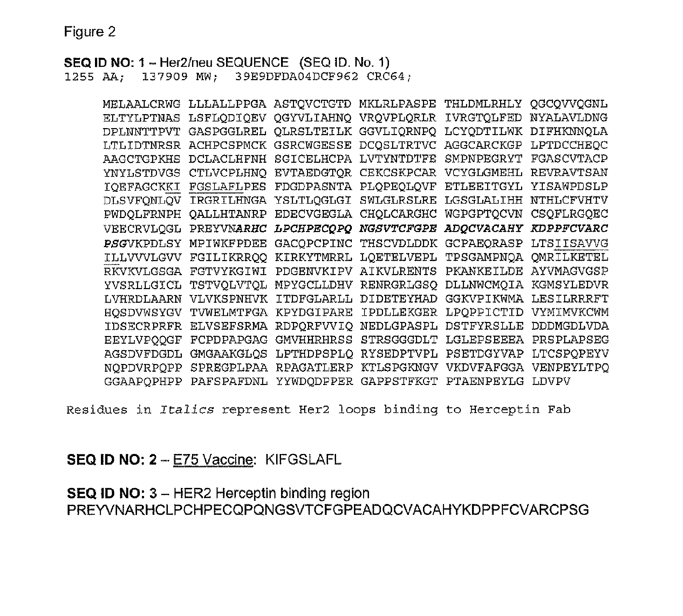

In an embodiment, the peptide comprises a contiguous amino acid sequence of at least 7 amino acid residues e.g. at least 8 amino acid residues, which represents a linear or non-linear epitope of an antibody binding region of the HER2/neu protein. In one embodiment, the antibody binding region is the region to which the antibody used in combination with the peptide binds. In one embodiment, the peptide represents a linear epitope present in an antibody binding region of the HER2/neu protein. In one embodiment, the peptide has a contiguous amino acid sequence of at least 7 amino acid residues that are comprised as a contiguous sequence within an antibody binding region of the HER2/neu protein. In one embodiment, the peptide has an amino acid sequence which represents a contiguous sequence in the HER2/neu protein which is proximal to the antibody binding region of the HER2/neu protein. Thus, in one embodiment, the peptide represents a sequence which is no more than about 1, 2, 3, 4, 5, 6, 7, 8, 9 or 10 amino acid residues from the antibody binding region of the HER2/neu protein. In one embodiment, the antibody binding region is the region of the HER2/neu protein to which trastuzumab binds. The region of the HER2/neu protein to which trastuzumab binds is shown in SEQ ID. No. 3 and Figure 2.

In one embodiment, the peptide is obtainable by a method comprising:
(a) binding an anti HER2/neu antibody to a Her/neu protein to form a complex;
(b) subjecting the complex to proteasome digestion;
(c) obtaining the digestion product comprising peptides; and
(d) identifying an immunogenic peptide sequence from the digestion products.

In one embodiment, the peptide is identifiable by the methods disclosed herein.

In one embodiment, the peptide may consist of from 7 to 30 amino acid residues, optionally between 7 and 20 amino acid residues and further optionally between 7 and 15 amino acid residues, and further optionally from 7 to 12 amino acid residues. In one embodiment, the peptide consists of between 8 and 10 amino acid residues e.g. 9 amino acid residues.

In one embodiment, the peptide is not E75 (SEQ. ID. No. 2). In an embodiment, the peptide is independently selected from (1) E75, which has the amino acid sequence KIFGSLAFL (SEQ ID NO: 2); and (2) GP2, which has an amino acid sequence which corresponds to amino acid residues 654-662 of the HER2/Neu protein (as shown in SEQ ID. No. 19). In one embodiment the peptide is not GP2. In one embodiment, the peptide is a GP2 variant, e.g. a variant which has the amino acid sequence shown in SEQ ID. No. 20.

In one embodiment, the peptide has an amino acid sequence independently selected from SEQ ID NO: 4, 5, 6, 7, 8, 9, 10, 11, 12, 13, 14, 15, 16, 17 and 18. In one embodiment, the peptide consists of the amino acid sequence shown in SEQ ID. No. 4. In one embodiment, the peptide consists of the amino acid sequence shown in SEQ ID. No. 5. In one embodiment, the peptide consists of the amino acid sequence shown in SEQ ID. No. 6. In one embodiment, the peptide consists of the amino acid sequence shown in SEQ ID. No. 7. In one embodiment, the peptide consists of the amino acid sequence shown in SEQ ID. No. 8. In one embodiment, the peptide consists of the amino acid sequence shown in SEQ ID. No. 9. In one embodiment, the peptide consists of the amino acid sequence shown in SEQ ID. No. 10. In one embodiment, the peptide consists of the amino acid sequence shown in SEQ ID. No. 11. In one embodiment, the peptide consists of the amino acid sequence shown in SEQ ID. No. 12. In one embodiment, the peptide consists of the amino acid sequence shown in SEQ ID. No. 13. In one embodiment, the peptide consists of the amino acid sequence shown in SEQ ID. No. 14, SEQ or ID. No. 15 or SEQ. ID. No. 16 or SEQ. ID. No. 17 or SEQ. ID. No. 18.

In one embodiment, the peptide may be administered in combination with a cytokine, e.g. interferon-α, IL-2, IL-12 or GM-CSF. In one embodiment, more than one peptide as described herein may be administered to the patient. Such administration may be simultaneous, sequential or separate.

In one aspect of the present invention, there is provided an anti-HER2/neu antibody for use to treat a cancer e.g. a cancer associated with over-expression of a HER2/neu protein, wherein the anti-HER2/neu antibody is for separate, sequential or simultaneous administration with one or more peptides consisting of a contiguous amino acid sequence which is comprised within the HER2/neu protein or a variant peptide thereof. In one embodiment, the peptide is as described herein. Thus, in one embodiment, the peptide represents a sequence comprised within or proximal to the region of the HER2/neu protein to which the antibody binds. In an embodiment, the term "proximal" relates to a peptide which has a sequence which represents a sequence no more than for example 1, 2, 3, 4, 5, 6, 7, 8, 9 or 10 amino acids from the antibody binding region of the HER2/neu protein. In one embodiment, the antibody is trastuzumab. In an embodiment, the cancer is breast cancer. In one embodiment, the cancer is ovarian cancer, prostate cancer or stomach cancer.

The present disclosure also includes a method comprising, in any order: administering a therapeutically effective amount of an antibody to the patient, wherein the antibody is trastuzumab; and administering a therapeutically effective amount of a peptide as disclosed herein to the patient. In one embodiment, the peptide is administered before, after or at the same time as administration of the antibody. In one embodiment, administration of the peptide and administration of the antibody have a synergistic effect on the patient. In one embodiment, the patient is a breast cancer patient.

In one aspect of the present invention, there is provided a peptide having an amino acid sequence shown in any of SEQ. ID. No. 4 to 18, e.g. SEQ. ID. No. 4, SEQ. ID. No. 5, SEQ. ID. No. 6, SEQ. ID. No. 7, SEQ. ID. No. 8, SEQ. ID. No. 9, SEQ. ID. No. 10, SEQ. ID. No. 11, SEQ. ID. No. 12, SEQ. ID. No. 13, SEQ. ID. No. 14, SEQ ID. No. 15. SEQ. ID. No. 16. SEQ ID. No. 17 and SEQ ID. No. 18 for use in treating cancer. In one embodiment, the peptide for use in combination (simultaneously, sequentially or separately) with at least one other anti-cancer therapy e.g. is for use in combination with an anti HER2/neu medicament. As shown in the Figures, cells stimulated with 2G-577 peptide (SEQ ID. No. 4) can lyse tumour cells.

The present invention also envisages the use of the peptide as defined herein in the manufacture of a medicament for the treatment of cancer, wherein the peptide is for administration sequentially, simultaneously or separately with an anti-HER2/neu antibody. In one embodiment, the antibody is trastuzumab. In an embodiment, the medicament is for the treatment of breast cancer e.g. HER2 overexpressing breast cancer e.g. HER2 over expressing node positive breast cancer. In one embodiment, the medicament is for stomach cancer, prostate cancer or ovarian cancer.

In one aspect of the invention, there is provided use of a peptide which has an amino acid sequence of (1) at least 8 contiguous amino acid residues of a HER2/neu protein or (2) a variant thereof, for the manufacture of a medicament for the treatment of cancer. In one embodiment, the peptide is selected from SEQ ID. No 4, 5, 6, 7, 8, 9, 10, 11, 12, 13, 14, 15, 16, 17 and 18.

### Detailed Description and Examples

Treatments for complex diseases involving the immune system and immune responses caused by endogenous self antigens and/or foreign antigens that are involved in producing autoimmune antibody are extremely difficult to discover. Antigens involved with such diseases are either foreign or self antigens (or both). Administration of foreign antigens for passive immunization can result in serum sickness-like immune complex diseases. Also, reactive T cells capable of recognizing self peptides are typically deleted or processed and destroyed. These peptides, generated and displayed under normal and constitutive conditions are degraded by cell protein degradation machinery resulting in the absence of an immune response.

A simple method for identification of immunogenic regions of self-proteins and other proteins and molecules involved in the binding interactions with polyclonal and monoclonal antibodies has been surprisingly discovered. From this method, new and unique epitopes are generated that are presented in the presence of bound ligands, such as, preferably antibodies. Once these immunogenic regions are identified, vaccines comprising the antigen, modifications of the antigen, or antibodies specifically reactive to the antigen or the modified antigen can be prepared. Thus, the present invention makes vaccine peptide discovery manageable, and allows for the specific generation of unique immunogenic peptides from self-tumor associated proteins that can be induced or generated for specific expression in the presence of the antibody, allowing for vaccine and/or novel combination therapy.

The term "epitope" as used herein includes the site on an antigen recognized by an agent as determined by the specificity of the amino acid sequence. Two agents are said to bind to the same epitope if each competitively inhibits (blocks) binding of the other to the antigen as measured in a competitive binding assay (see, e.g., Junghans et al., Cancer Res. 50:1495-1502, 1990). Alternatively, two antibodies have the same epitope if most amino acid mutations in the antigen that reduce or eliminate binding of one antibody reduce or eliminate binding of the other. Two antibodies are said to have overlapping epitopes if each partially inhibits binding of the other to the antigen, and/or if some amino acid mutations that reduce or eliminate binding of one antibody reduce or eliminate binding of the other.

The invention is described more fully herein and refers to many preferred embodiments. This invention, however, should not be construed as limited to those embodiments.

The proteasome is a multi-subunit complex with proteolytic cleavage activities that result in the generation of a wide variety of peptides from proteins. The susceptibility of a given protein to the proteolytic activities of the proteasome is dependent upon the various primary, secondary and tertiary structural and post-translational modifications that take place within the proteasome. These activities expose certain sequences or regions of the protein and not others, to the system.

In one embodiment of the invention, cancer cells are induced to express immunogenic peptides from unique or self tumor-specific antigens to stimulate anti-tumor immune responses for immunotherapy. Normally these peptides are not generated from self-proteins. Binding of antibodies (or other molecules) to the sites normally accessible and processed by proteasomes alters the pattern of accessibility and the resulting proteolytic cleavage pattern by the proteasome. Such alteration of the site results in generation of novel peptides that may be intrinsically immunogenic because they have not been previously expressed and displayed for the deletion of immuno-reactive T cells.

In one preferred embodiment, a unique immunogenic region of the HER-2/neu was identified. HER-2/neu is an over-expressed oncogenic protein. Conventional vaccine strategies, normally effective in immunizing patients, did not work with a "self-protein" such as HER-2/neu. Tolerance to self-proteins may only be directed to dominant epitopes of the protein and not the entire protein. Therefore, immunization to just a specific protein fragment, and not the entire protein, alleviates this problem. This specific protein fragment is located with within the sequence directly involved with antibody-binding interaction or in the proximity of that region.

After identifying this restricted, shorter peptide sequence, the sequence is subjected to algorithms to identify likely functionally active or target sequences or regions. Running algorithms on this segment, as opposed to the whole segment, provides a manageable set of peptides to test as candidates for vaccine development. In the past, computer-driven tests were run on the entire sequence consuming much time, money and effort. The algorithms searched the amino acid sequence provided for characteristic immune response *in vitro.* Regions of the proteins identified as containing epitopes may be useful as a vaccine.

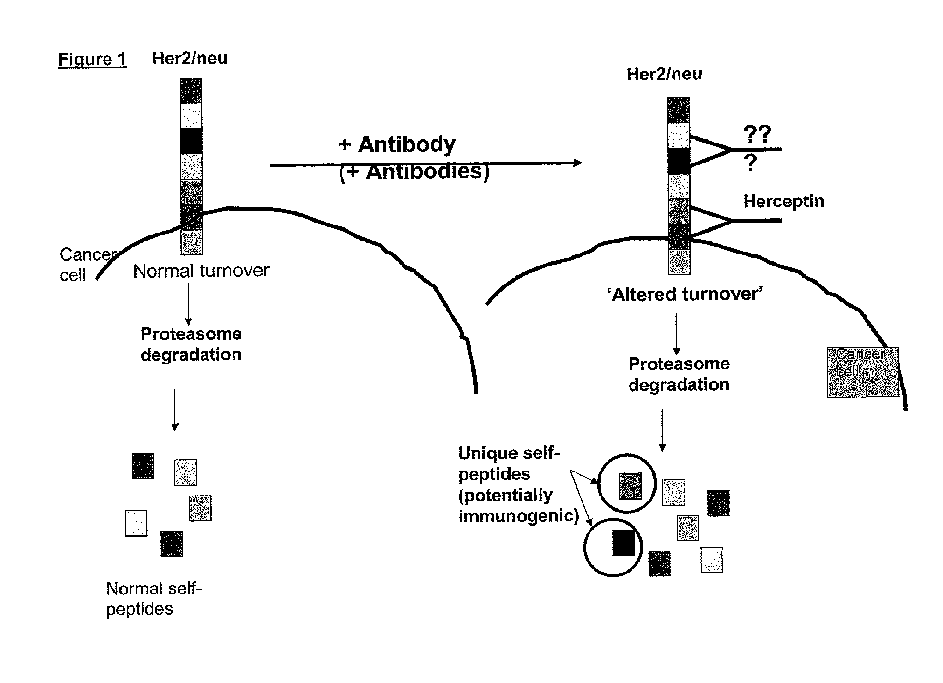

Next, treatment of the tumor cells with the antibody against the identified peptide sequence was performed. Inducibility of the altered turnover and subsequent generation of the newly identified peptides only in the tumor cells and only in the presence of the antibody gave it a specific targeting and triggering feature that was controllable. See Figure 1. An antibody booster can be given to increase the peptide-specific cytotoxic T cell response. In cases where the antibody already existed, discovery of the peptide only was sufficient for triggering the specific immune response.

The method to generate new and unique epitopes included the identification of immunogenic peptides from regions of proteins and molecules involved in the binding interactions with most any ligand including, but not limited to polyclonal and monoclonal antibodies, receptors, ligands and any molecule with high affinity to the antigen. These new epitopes were presented to antigen-processing cells in the presence of bound ligand. In certain cases, binding protected the epitope or a portion of the epitope revealing the immunogenic portion of the antigen. These immunogenic regions become new and uniquely enhanced targets for recognition by the immune system and for use in modulation of the immune responses for the treatment of disease states and immunotherapy either by themselves, an antibody vaccines, or when used in combination with other molecules or antibodies. These antibody-generated immunogenic epitopes (AGIE)/antibody-bound immunogenic epitopes (ABIE) were useful for stimulating immune response in cancer and infectious diseases. They can also suppress immune responses in autoimmune diseases and transplantation based upon the activity of the antibody in modulating the immune response in the direction of upregulation or down regulation.

Peptides varying in length including single chain antigen-binding polypeptides that can specifically interact, protect and/or modulate any given epitope such that it results in specifically protecting and/or enhancing the preservation and presentation of the epitope when subjected to the various proteolytic activities of the antigen processing machinery of the proteasome and immunoproteasome subunits and complexes.

Another embodiment of this invention comprises a combination of specific antibodies and/or treatment with other molecules that involve the same regions of the peptide sequences and targets unique populations of cells. This technology allows for the development of novel vaccines made of antibodies and/or antigen-binding fragments Fab or F(ab)'2-fragments bound to specific length peptides that contain immunogenic epitopes(s) of interest that are specifically processed and presented.

Specific epitopes of proteins may be protected, both intracellular and extracellular, that are normally destroyed by antigen processing machinery of proteasomes and immunoproteasomes by single chain antibodies and/or peptides that can specifically bind to selected site sequences so that these epitopes become novel targets that are developed as unique vaccines for the specific recognition of cancer cells. HSPs that are released during inflammatory processes perform similar activities and enhance immune responses because they were made to bind to many different peptide sequences non-covalently.

Another preferred embodiment of the invention is directed to a method for the identification of highly immunogenic peptides. These peptides are used to enhance or suppress immune responses from normal cell proteins that are not processed and presented naturally because of the constitutive destruction of these epitopes by the normal activities of the proteasome and immunoproteasome complexes. These peptides are used for generation for specific antibodies that bind and generate the novel presentation of these epitopes for recognition by the immune system.

In a preferred example, a large pool of immunoreactive T cells that are present or may be generated that are capable of reacting with these inert/naturally non-existent epitopes, but are not utilized and are considered "wasteful" because these epitopes are not being generated normally. This allows access to the pools of unused T cells for specifically directed therapeutic benefits. Again, computer-driven algorithms are run. Upon identification of the peptide sequences involved with antibody-binding interaction, these peptides are used to immunize animals and/or patients to generate specific antibodies that bind the protein and generate these novel peptides for subsequent immune recognition and response.

Another embodiment of the invention is directed to a direct method of binding antibody or antibodies to the protein molecules and/or polypeptide regions and subjecting these bound and unbound/native complexes to ex vivo or in vitro to all forms of the proteasome machinery. Proteolytic digestion or cleavage products are obtained to observe differential yield of peptides for use in vaccine development. However, there is a drawback to this method in that it is not fully representative of all "mechanisms and proteolytic activities" that may occur in vivo within the cell itself, thereby limiting application. Such an approach however, is useful for the proof of concept in systems where it provides clean reproducible and predictable results, e.g. using various individual components of proteasomes and combinations thereof and then observing peptide patterns by mass spectroscopy analysis. It is also useful for the definition of peptide patterns generated in the presence and/or absence of proteasome inhibitors with and without bound antibody or antibodies.

Another embodiment of the present invention involves application of this approach for identification of patient-specific treatment. Optimal antibody choices are made for the patient based upon patient's personal pre-existing immuno-reactive precursors to the AGIE/ABIE. First, PBMC (peripheral blood mononuclear cells) are obtained from patient and culture in presence or absence of cytokines (eg IL-2, IL-7, and IL-15) in the presence of FCS (fetal calf serum) or autologous serum. After optimal culture and expansion during a set number of days, the cells are tested in cytotoxicity assays against tumor cell lines that have been pre-incubated in the presence and absence of specific antibodies or antibody combination(s). Datasets from responses seen specifically to presence of antibodies and those where individual antibodies do not yield a response, but the combinations that do are noted. The epitopes/vaccine peptides predicted or defined by the binding-sites of the antibodies are tested for fine reactivities in determination of which vaccines to use on patients.

Another embodiment of the invention is directed to identifying antigens that are not expressed on the cell surface or for parts of protein that are always cytoplasmic or for proteins that are completely intracellular/intranuclear or cytoplasmic (e.g. p53, telomerase, etc.). Methods for expressing the "modulating" antibody or antibodies as endogenous proteins, so that they are able to bind these proteins intracellularly and modulate their processing and presentation from within. The endogenous expression methods included recombinant DNA expression systems as well as viral/bacterial vector delivery and expression systems.

Another embodiment of the invention is directed to "shuttling" in the antibodies or single-chain antigen-binding fragment encapsulated within various delivery systems to include liposomes, micelles, nanoparticles and others.

Generation of "protective" or "suppressive" antibody preparations or combination capable of being administered passively for treatment of disease similar to the gamma-globulin shots were generated as polyclonal preps or as specific combinations of selected antibodies against one or more tumor or tissue specific antigens for that particular disease or condition.

The present invention is not restricted to HLA-A2 or class I peptides because one or more longer-length peptides from a known region or sequence can be used for the generation of AGIE/ABIE by the antibody or antibodies in question. The benefit is not having to be restricted by only specific or limited HLA types (Class I and II) in terms of patients that can be treated. Furthermore, although the majority of Class I and Class II peptides are derived from the processing of endogenous and exogenous proteins respectively, the well described and accepted mechanisms of "cross-presentation" allows for the presentation of all sources or peptides on both classes of MHC molecules.

In another embodiment of the invention, the invention is used for identifying inducible vaccine responses by "discovering" the MAb/s that will generate AGIE/ABIE. This is achieved by first screening 10-mer or 20-mer consecutive or overlapping peptides from antigen for the highest precursor T-cell responses present in cancer and/or normal individuals. These "ultra-immunogenic" peptides are then used for generation of MAb in mice. These MAbs are then tested for ability to generate AGIE/ABIE specific responses in MAb-treated targets. MAbs that are promising are then humanized for therapeutic purposes. To ensure involvement of T cell specific immune responses the promising Fab' fragments are first used to eliminate purely antibody-dependent cell-mediated cytotoxicity (ADCC)-mediated activity.

Another way of specifically identifying peptides capable of generating antibodies that induce AGIE/ABIE that are most protective is by the method of screening serum sample from high-risk cancer individuals (those with family history of cancer, e.g. smokers who do and do not get lung cancer, patients who are 'cured') who do and do not develop cancer (or from progressor vs non-progressors in the case of AIDS) to look for Ab responses against the overlapping peptides from specific well characterized or defined tumor antigens (HER2/neu, prostate specific antigen or PSA, prostate specific membrane antigen or PSMA, Tyrosinase, melanoma Ags, etc.) that are present in the protected individuals or present in fully recovered/cured cancer patients. Based on the specific Ab responses that are found to be uniquely present or predominantly present in the 'protected' individuals, peptides are targeted for generation or 'discovering' of MAb that generated AGIE/ABIE. All descriptions herein were done for both Class I and Class II epitopes and responses.

In a preferred embodiment, data of appropriate combinations of antibodies from published sources with well documented 'pre-existing' corresponding CD4-helper and CD8 CTL-specific responses is used for developing a vaccine for the AIDS virus and other immunotherapy treatment using the approach from the present invention. The HIV Molecular Immunology Database provided by the National Institute of Allergy and Infectious Diseases at www,hiv,lanl.gov offers a current comprehensive listing of defined HIV epitopes. The website also includes the exact epitope and binding site of all known monoclonal and polyclonal antibodies to the protein sequences as well as neutralization activity.

The present invention provides enhanced lytic activity of antibody-treated tumor cells by vaccines identified from the binding sites of these antibodies. The present invention also provides a novel indication for antibodies already in clinical use for cancer treatment as combination therapy furthering development of vaccine treatment discovery.

In one embodiment, the peptide as described herein is for use in treating a HER2/neu positive cancer. The peptide may be for use with an anti HER2/neu antibody. In one embodiment, the antibody is trastuzumab. Trastuzumab is disclosed in US5,821, 337, the contents of which are incorporated herein by reference.

Also encompassed by the present invention are antibody fragments, e.g. an anti-HER2/neu Fab fragment. Naturally within the scope of the invention are antibodies or fragments which are monoclonal, polyclonal, chimeric, human, or humanized.

An antibody and immunologically active portions thereof, for instance, are typically molecules that contain an antigen binding site which specifically binds (immunoreacts with) an antigen. A naturally occurring antibody (for example, IgG) includes four polypeptide chains, two heavy (H) chains and two light (L) chains interconnected by disulfide bonds. The two heavy chains are linked to each other by disulfide bonds and each heavy chain is linked to a light chain by a disulfide bond. There are two types of light chain, lambda (λ) and kappa (k). There are five main heavy chain classes (or isotypes) which determine the functional activity of an antibody molecule: IgM, IgD, IgG, IgA and IgE. Full-length immunoglobulin light chains are generally about 25 Kd or 214 amino acids in length. Full-length immunoglobulin heavy chains are generally about 50 Kd or 446 amino acid in length. Light chains are encoded by a variable region gene at the NH2-terminus (about 110 amino acids in length) and a kappa or lambda constant region gene at the COOH--terminus. Heavy chains are similarly encoded by a variable region gene (about 116 amino acids in length) and one of the other constant region genes.

The basic structural unit of an antibody is generally a tetramer that consists of two identical pairs of immunoglobulin chains, each pair having one light and one heavy chain. In each pair, the light and heavy chain variable regions bind to an antigen, and the constant regions mediate effector functions. Immunoglobulins also exist in a variety of other forms including, for example, Fv, Fab, and (Fab')₂, as well as bifunctional hybrid antibodies and single chains (e.g., Lanzavecchia et al., Eur. J. Immunol. 17:105, 1987; Huston et al., Proc. Natl. Acad. Sci. U.S.A., 85:5879-5883, 1988; Bird et al., Science 242:423-426, 1988; Hood et al., Immunology, Benjamin, N.Y., 2nd ed., 1984; Hunkapiller and Hood, Nature 323:15-16, 1986).

Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, transplacental mobility, complement binding, and binding to Fc receptors. An immunoglobulin light or heavy chain variable region includes a framework region interrupted by three hypervariable regions, also called complementarity determining regions (CDR's) (see, Sequences of Proteins of Immunological Interest, E. Kabat et al., U.S. Department of Health and Human Services, 1983). As noted above, the CDRs are primarily responsible for binding to an epitope of an antigen. The specificity of the antibody resides in the structural complementarity between the antibody combining site and the antigenic determinant.

A monoclonal antibody is produced by a single clone of B-lymphocytes or by a cell into which the light and heavy chain genes of a single antibody have been transfected. Monoclonal antibodies are produced by methods known to those of skill in the art, for instance by making hybrid antibody-forming cells from a fusion of myeloma cells with immune spleen cells. Generally, a monoclonal antibody is produced by a specific hybridoma cell, or a progeny of the hybridoma cell propaged in culture. A hybridoma or other cell producing an antibody may be subject to genetic mutation or other changes, which may or may not alter the binding specificity of antibodies produced.

The antibody may be a chimeric antibody. Chimeric antibodies are antibodies whose light and heavy chain genes have been constructed, typically by genetic engineering, from immunoglobulin variable and constant region genes belonging to different species. For example, the variable segments of the genes from a mouse monoclonal antibody can be joined to human constant segments, such as kappa and gamma 1 or gamma 3. Methods of making chimeric antibodies are well known in the art, e.g., see U.S. Patent No. 5,807,715, which is herein incorporated by reference.

In one embodiment, the anti-HER2/neu antibody may be a humanized antibody or fragment thereof. A "humanized" immunoglobulin is an immunoglobulin including a human framework region and one or more CDRs from a non-human (such as a mouse, rat, or synthetic) immunoglobulin. The non-human immunoglobulin providing the CDRs is termed a "donor" and the human immunoglobulin providing the framework is termed an "acceptor." In one embodiment, all the CDRs are from the donor immunoglobulin in a humanized immunoglobulin. Constant regions need not be present, but if they are, they must be substantially identical to human immunoglobulin constant regions, i.e., at least about 85-90%, such as about 95% or more identical. Hence, all parts of a humanized immunoglobulin, except possibly the CDRs, are substantially identical to corresponding parts of natural human immunoglobulin sequences. A humanized antibody binds to the same antigen as the donor antibody that provides the CDRs. The acceptor framework of a humanized immunoglobulin or antibody may have a limited number of substitutions by amino acids taken from the donor framework. Humanized or other monoclonal antibodies can have additional conservative amino acid substitutions which have substantially no effect on antigen binding or other immunoglobulin functions. Exemplary conservative substitutions can be found in for example U.S. Patent No. 5,585,089, which is incorporated herein by reference. Humanized immunoglobulins can be constructed by means of genetic engineering, e.g., see U.S. Patent No. 5,225,539 and U.S. Patent No. 5,585,089, which are herein incorporated by reference.

In one embodiment, the antibody is a human antibody. A human antibody is an antibody wherein the light and heavy chain genes are of human origin. Human antibodies can be generated using methods known in the art. Human antibodies can be produced by immortalizing a human B cell secreting the antibody of interest. Immortalization can be accomplished, for example, by EBV infection or by fusing a human B cell with a myeloma or hybridoma cell to produce a trioma cell. Human antibodies can also be produced by phage display methods (see, e.g., Dower et al., PCT Publication No. WO91/17271; McCafferty et al., PCT Publication No. WO92/001047; and Winter, PCT Publication No. WO92/20791, which are herein incorporated by reference), or selected from a human combinatorial monoclonal antibody library (see the Morphosys website). Human antibodies can also be prepared by using transgenic animals carrying a human immunoglobulin gene (e.g., see Lonberg et al., PCT Publication No. WO93/12227; and Kucherlapati, PCT Publication No. WO91/10741, which are herein incorporated by reference).

As mentioned previously, the antibody may be an antibody fragment. Various fragments of antibodies have been defined, including Fab, (Fab')₂, Fv, dsFV and single-chain Fv (scFv) which have specific antigen binding. These antibody fragments are defined as follows: (1) Fab, the fragment that contains a monovalent antigen-binding fragment of an antibody molecule produced by digestion of whole antibody with the enzyme papain to yield an intact light chain and a portion of one heavy chain or equivalently by genetic engineering; (2) Fab', the fragment of an antibody molecule obtained by treating whole antibody with pepsin, followed by reduction, to yield an intact light chain and a portion of the heavy chain; two Fab' fragments are obtained per antibody molecule; (3) (Fab')₂, the fragment of the antibody obtained by treating whole antibody with the enzyme pepsin without subsequent reduction or equivalently by genetic engineering; (4) F(Ab')₂, a dimer of two Fab' fragments held together by disulfide bonds; (5) Fv, a genetically engineered fragment containing the variable region of the light chain and the variable region of the heavy chain expressed as two chains; dsFV, which is the variable region of the light chain and the variable region of the heavy chain linked by disulfide bonds and (6) single chain antibody ("SCA"), a genetically engineered molecule containing the variable region of the light chain, the variable region of the heavy chain, linked by a suitable polypeptide linker as a genetically fused single chain molecule. Single chain antibodies may also be referred to as single chain variable fragments (scFv). Methods of making these fragments are routine in the art.

Reference is made to the numbering scheme from Kabat, E. A., et al., Sequences of Proteins of Immunological Interest (National Institutes of Health, Bethesda, MD (1987) and (1991). In these compendiums, Kabat lists many amino acid sequences for antibodies for each subclass, and lists the most commonly occurring amino acid for each residue position in that subclass. Kabat uses a method for assigning a residue number to each amino acid in a listed sequence, and this method for assigning residue numbers has become standard in the field. CDR and FR residues are also determined according to a structural definition (as in Chothia and Lesk, J. Mol. Biol. 196:901-917 (1987). Where these two methods result in slightly different identifications of a CDR, the structural definition is preferred, but the residues identified by the sequence definition method are considered important FR residues for determination of which framework residues to import into a consensus sequence.

In one embodiment, the antibody is trastuzumab, which is marketed under the trademark Herceptin®.

In one embodiment, the peptides of the present invention are for administration in combination with an anti-HER2/neu antibody. Such administration may be sequential, simultaneous or separate.

In one embodiment, the method comprises the combined use of a peptide as described herein and an anti-HER2/neu antibody to treat cancer e.g. a cancer which is associated with overexpression of the HER2/neu protein. "Combined use" and "combination" in the context of the invention means the simultaneous, sequential or separate administration of the peptide as defined herein on the one hand and of the anti HER2/neu antibody.

In one embodiment, the peptide and the antibody may be administered simultaneously. Simultaneous administration includes for example (1) the simultaneous uptake of two separate dosage forms containing the peptide in one of the dosage forms and the antibody in the other dosage form or (2) pharmaceutical compositions containing both active ingredients in one single dosage form (fixed unit dose form).

"Combined use" and "combination" in the context of the invention also includes a pharmaceutical product comprising both the peptide and the antibody as discrete separate dosage forms, in separate containers or e. g. in blisters containing both types of drugs in discrete solid dosage units, e.g. in a form in which the dosage units which have to be taken together or which have to be taken within one day are grouped together in a manner which is convenient for the patient. Said pharmaceutical product itself or as a part of a kit may contain instructions for the simultaneous, sequential or separate administration of the discrete separate dosage units, to a patient in need thereof.

Sequential administration in the context of the invention includes the administration of the peptide as defined herein on the one hand and of the anti-HER2/neu antibody on the other hand in separate dosage forms within less than 12 hours, e.g. within 5 hours or less, for example less than one hour, or e.g. within 30, 25, 15, 5 minutes or less.

In one embodiment, the peptide and the antibody may be administered separately. Separate administration within the context of the invention means the administration of the peptide on the one hand and of the antibody on the other hand in separate dosage forms spaced apart by 12 hours or more e.g. by 18 hours or 24hours.

The peptides and/or the antibody may normally be administered orally, intravenously, subcutaneously, buccally, rectally, dermally, nasally, tracheally, bronchially, by any other parenteral route, as an oral or nasal spray or via inhalation. The peptides may be administered in the form of pharmaceutical preparations in a pharmaceutical acceptable dosage form. Depending upon the disorder and patient to be treated and the route of administration, the compositions may be administered at varying doses. Preferably, the antibody is for intravenous administration via infusion.

Typically, therefore, the pharmaceutical compounds of the invention may be administered orally or parenterally ("parenterally" as used herein, refers to modes of administration which include intravenous, intradermally, intramuscular, intraperitoneal, intrasternal, subcutaneous and intraarticular injection and infusion of which intavenous is most preferred) to a host to obtain a desired effect. In the case of larger animals, such as humans, the compounds may be administered alone or as compositions in combination with pharmaceutical acceptable diluents, excipients or carriers.

If a peptide or an antibody of the invention is to be administered to an individual, it is particularly at least 80% pure, more preferably it is at least 90% pure, even more preferably it is at least 95% pure and free of pyrogens and other contaminants. In this context, the percent purity is calculated as a weight percent of the total protein content of the preparation, and does not include constituents which are deliberately added to the composition after the agent is purified.

Actual dosage levels of active ingredients in the pharmaceutical compositions of this invention may be varied so as to obtain an amount of the active agent (s) that is effective to achieve the desired therapeutic response for a particular patient, compositions, and mode of administration. The selected dosage level will depend upon the activity of the particular agent, the route of administration, the severity of the condition being treated and the condition and prior medical history of the patient being treated. However, it is within the skill of the art to start doses of the agent at levels lower than required for to achieve the desired therapeutic effect and to gradually increase the dosage until the desired effect is achieved. In one embodiment, the anti-HER2/neu antibody may be administered over a period of between 1 to 2 hours e.g. 90 minutes. The antibody may be administered at a concentration of from 2mg/kg to about 10mg/kg e.g. 2mg/kg, 4mg/kg, 6mg/kg or 8mg/kg. The concentration of the antibody may vary over a course of treatment of the patient.

The terms "treatment", "treating" and the like are used herein to generally mean obtaining a desired pharmacological and/or physiological effect. The effect may be prophylactic in terms of completely or partially preventing a disease or symptom thereof and/or may be therapeutic in terms of partially or completely curing a disease and/or adverse effect attributed to the disease. The term "treatment" as used herein covers any treatment of a disease in a mammal, particularly a human, and includes: (a) preventing the disease from occurring in a subject which may be predisposed to the disease; (b) inhibiting the disease, i.e. arresting its development; or (c) relieving the disease, i.e, causing regression of the disease. The term "treatment" may also include alleviating symptoms of a disorder.

Further embodiments of the present invention will be apparent from the description that follows and with the items [1] to [43] below representing preferred embodiments
[1] A peptide which has an amino acid sequence of (1) at least 8 contiguous amino acid residues of a HER2/neu protein or (2) a variant thereof, for administration in combination with an anti HER2/neu protein for the treatment of a cancer, wherein said administration is sequentially, simultaneously or separate and wherein optionally the cancer is associated with over-expression of the HER2/neu protein.
[2] A peptide according to [1], which represents an amino acid sequence that is comprised within the antibody binding region of the HER2/neu protein.
[3] A peptide according to [1] or [2], wherein the variant comprises a mutation e.g. substitution, for improved binding to MHC claim I molecules e.g. a substitution of the amino acid residues at position 2 to leucine or methionine and/or substitution to valine at position 9 for HLA-A2.
[4] A peptide according to [3], which comprises an amino acid substitution at position 2 and/or position 9 of the peptide.
[5] A peptide according to [3], wherein the variant comprises an amino acid substitution which replaces the amino acid residue at position 2 with (1) a glycine residue; (2) leucine residue (3) methionine or (4) valine residue.
[6] A peptide according to [4], which comprises a mutation at amino acid residue 9.
[7] A peptide according to [6], wherein the mutation is an amino acid substitution which replaces the amino acid residue at position 9 with a valine residue.
[8] A peptide according to any of [1] to [7], wherein the variant comprises an additional group e.g. a methylene group at position 4.
[9] A peptide according to [1], which has a contiguous amino acid sequence of at least 7 amino acid residues which represent a linear or non-linear epitope of an antibody binding region of the HER2/neu protein.
[10] A peptide according to [9], which has a contiguous amino acid sequence of at least 7 amino acid residues that are comprised as a contiguous sequence within the region of the HER2/neu protein to which the antibody binds.
[11] A peptide according to any preceding item, which is obtainable by a method comprising:
   (a) binding an anti H ER2/neu antibody to a Her/neu protein to form a complex;
   (b) subjecting the complex to proteasome digestion;
   (c) obtaining the digestion product comprising peptides; and
   (d) identifying an immunogenic peptide sequence from the digestion products.
[12] A peptide according to any preceding item, which consists of between 7 to 30 amino acid residues, and optionally between 7 and 20 amino acid residues and further optionally between 7 and 15 amino acid residues, and further optionally 7 and 12 amino acid residues.
[13] A peptide according to [12], which consists of between 8 and 10 amino acid residues e.g. 9 amino acid residues.
[14] A peptide according to any preceding item, wherein the peptide is not E75 (SEQ. ID. No. 2) and/or is not GP2.
[15] A peptide according to [1], which has the amino acid sequence KIFGSLAFL (SEQ ID NO: 2)
[16] A peptide according to [1], which is GP2 and has an amino acid sequence which corresponds to amino acid residues 654-662 of the HER2/Neu protein.
[17] A peptide according to [1], wherein the peptide has the amino acid sequence of SEQ ID NO: 4, 5, 6, 7, 8, 9, 10, 11, 12, 13, 14, 15, 16, 17, 18, 19 or 20.
[18] A peptide according to any preceding item, for use with a cytokine, wherein optionally said cytokine is GM-CSF.
[19] A therapeutically effective composition comprising a peptide that represents a portion of the sequence of HER2/neu antigen, wherein the peptide contains the sequence of SEQ ID NO: 2 (E75 = KIFGSLAFL), and further comprising an antibody that binds to an immunogenic region of the antigen.
[20] The composition of [19], wherein the peptide sequence is substantially within the immunogenic region of the antibody binding site.
[21] The composition of [20], wherein the antibody is trastuzumab.
[22] A vaccine comprising the composition of [21], which is therapeutically effective in the treatment of cancer.
[23] The vaccine of [22], wherein the cancer is breast cancer.
[24] A pharmaceutical composition for use in the treatment of cancer comprising a peptide that represents a portion of the sequence of HER2/neu antigen, wherein the peptide contains the sequence of SEQ ID NO: 2 (E75 = KIFGSLAFL), and further comprising an antibody that binds to an immunogenic region of the antigen, wherein the antibody is trastuzumab.
[25] A peptide which represents a portion of the sequence of HER2/neu antigen, and contains the sequence of SEQ ID NO: 2 (E75 = KIFGSLAFL) for treating a patient in a method comprising, in any order:
   a. administering a therapeutically effective amount of an antibody to the patient, wherein the antibody is trastuzumab; and
   b. administering a therapeutically effective amount of the peptide to the patient.
[26] The peptide of [25], wherein the peptide is administered before, after or at the sametime as administration of the antibody.
[27] The peptide of [26], wherein administration of the peptide and administration of the antibody have a synergistic effect on the patient.
[28] The peptide of [27], wherein the patient is a breast cancer patient.
[29] A therapeutically effective composition comprising a peptide that represents a portion of the sequence of HER2/neu antigen, wherein the peptide contains the sequence of SEQ ID NO: 19 (GP2 = IISAVVGIL) or SEQ ID NO: 20 (GP2' = IVSAVVGIL), and further comprising an antibody that binds to an immunogenic region of the antigen.
[30] The composition of [29], wherein the peptide sequence is substantially within the immunogenic region of the antibody binding site.
[31] The composition of [30], wherein the antibody is trastuzumab.
[32] A vaccine comprising the composition of [29], which is therapeutically effective in the treatment of cancer.
[33] The vaccine of [32], wherein the cancer is breast cancer.
[34] A pharmaceutical composition for use in the treatment of cancer comprising a peptide that represents a portion of the sequence of HER2/neu antigen, wherein the peptide contains the sequence of SEQ ID NO: 19 (GP2 = IISAVVGIL) or SEQ ID NO: 20 (GP2'= IVSAVVGIL), and further comprising an antibody that binds to an immunogenic region of the antigen, wherein the antibody is trastuzumab.
[35] A peptide which represents a portion of the sequence of HER2/neu antigen, and which contains the sequence of SEQ ID NO: 19 (GP2 = IISAVVGIL) or SEQ ID NO: 20 (GP2'=IVSAVVGIL) for use in treating a patient in a method comprising in any order:
   a. administering a therapeutically effective amount of an antibody to the patient, wherein the antibody is trastuzumab; and
   b. administering a therapeutically effective amount of the peptide to the patient.
[36] The method of [35], wherein the peptide is administered before, after or at the same time as administration of the antibody.
[37] The method of [36], wherein administration of the peptide and administration of the antibody have a synergistic effect on the patient.
[38] The method of [37], wherein the patient is a breast cancer patient.
[39] An anti-HER2/neu antibody for use to treat a cancer associated with over-expression of a HER2/neu protein, wherein the anti-HER2/neu antibody is for separate, sequential or simultaneous administration with one or more peptides consisting of a contiguous amino acid sequence which is comprised within the HER2/neu protein.
[40] An antibody according to [39], wherein the peptide is as in any of [7] and [17] to [38] or a permissible combination thereof.
[41] An antibody according to [39] or 40, wherein the antibody is trastuzumab.
[42] An antibody according to any of [39] to [41], wherein the cancer is breast cancer.
[43] An antibody according to any of [39] to [42], wherein the peptide represents a region of the HER2/neu protein to which the antibody binds.

### Examples

The binding of trastuzumab (Herceptin) to HER2/neu for the development of novel vaccines against HER2/neu-expressing tumors was studied. The Ab-binding site of trastuzumab (Tz) was analyzed for peptides that bind HLA-A2 and A3. A peptide Her577 (aa: 577-585) within the Ab-binding site on HER2/neu was identified, synthesized and tested. T2 HLA-stabilization assays were performed to confirm HLA-A2 binding activity of Her577 by flow cytometry. PBMC from 9 healthy donors were stimulated with Her577 and tested in ⁵¹Cr-release cytotoxicity (CTX) assays against 3 HER2/neu-expressing tumor cell lines. Her577-stimulated PBMC from 5 HLA-A2⁺ healthy donors were also tested in CTX with HER2/neu⁺ targets pre-treated with Tz. Simultaneous experiments were done with E75 and GP2, two other immunogenic peptides from HER2/neu.

The Her577 peptide bound HLA-A2 comparable to E75 and GP2 with mean fluorescence intensity values of 1275, 1151 and 682, respectively. The average specific CTX by Her577-stimulated cytotoxic T lymphocytes (CTL) against LNCaP, SKOV-3 and MCF-7 tumor cells was found to be similar to CTX achieved with E75- and GP2-stimulated CTL (% lysis; Table 1).

**Table 1**

| | LNCaP | SKOV-3 | MCF-7 |
|---|---|---|---|
| | (HLA-A2⁺) | (HLA-A3⁺) | (HLA-A2⁺) |
| | (n = 2) | (n = 2) | (n = 5) |
| Her577 | 30±3 | 49±28 | 45±3 |
| E75 | 30±1 | 51±20 | 51±4 |
| GP2 | 26±9 | 46±5 | 48±1 |

Pre-treatment with Tz at 50µg/ml for 24h increased specific CTX by Her577-stimulated CTL versus untreated MCF-7, SKOV-3 and LNCaP by 16%, 47% and 83%, respectively (P =0.19). A dose-dependent incremental increase in CTX against each tumor cell line was seen with 10 and 50µg/ml doses of Tz.

It can be concluded that Her577 is a newly described T cell-epitope from HER2/neu identified by using a novel method for the discovery of immunogenic peptide(s) for vaccine development by assessing the binding site of Tz. There is a potential for combination immunotherapy with therapeutic Ab and unique peptide vaccines derived from the Ab-binding site.

### Example 2

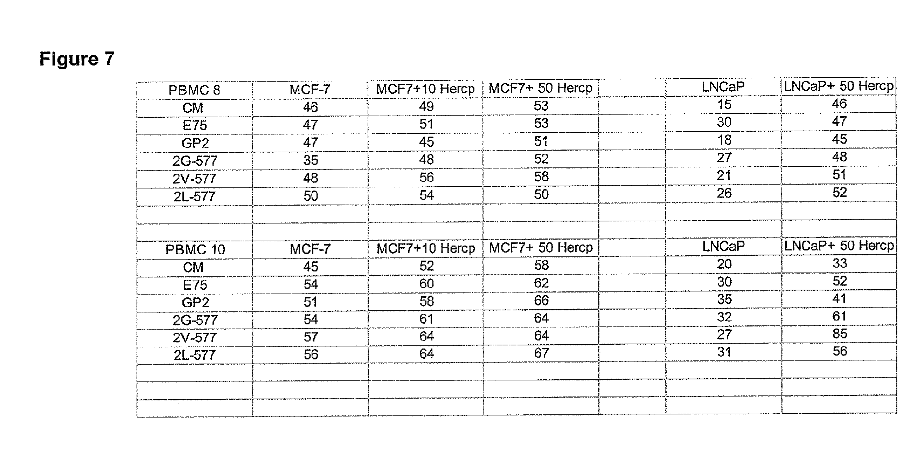

Cells are stimulated by 2G, 2V and 2L-577 peptides (2V and 2L-577 are peptides which have been mutated from the Her577 peptide at position 2 to replace glycine with valine and leucine respectively. The % lysis of Herceptin treated tumour targets by the cells is detected as shown in Figure 7.

### Example 3

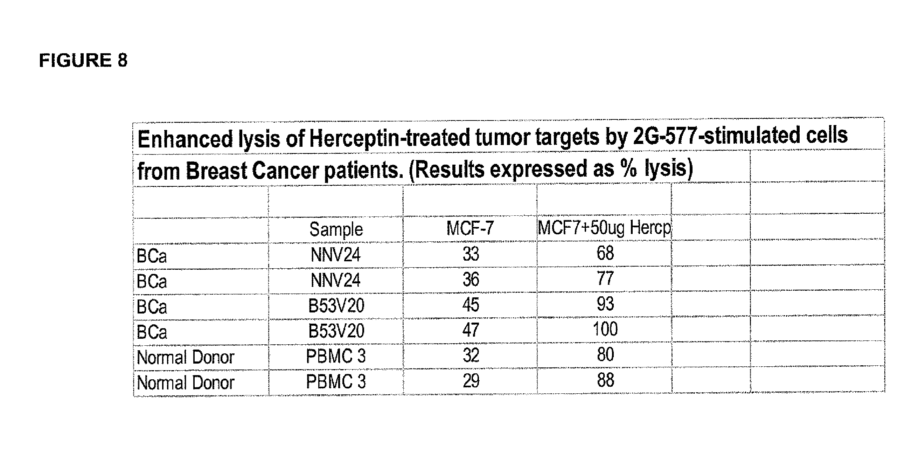

Cells taken from breast cancer patients are stimulated by 2G-577. The lysis of Herceptin-treated tumor targets is detected. The results are shown in Figure 8.

Other embodiments and uses of the invention will be apparent to those skilled in the art from consideration of the specification and practice of the invention disclosed herein. All references cited herein, including all patents and publications that are cited for any reason, are specifically and entirely incorporated by reference. The specification and examples should be considered exemplary only with the true scope and spirit of the invention embodied within the following claims.

## Claims

1. A peptide which has an amino acid sequence of (1) at least 8 contiguous amino acid residues of a HER2/neu protein or (2) a variant thereof, for administration in combination with an anti HER2/neu protein for the treatment of a cancer, wherein said administration is sequentially, simultaneously or separate and wherein optionally the cancer is associated with over-expression of the HER2/neu protein, wherein the peptide is not E75 (SEQ ID NO: 2) and/or is not GP2.

2. A peptide according to claim 1, which represents an amino acid sequence that is comprised within the antibody binding region of the HER2/neu protein.

3. A peptide according to claim 1 or claim 2,
i) wherein the variant comprises a mutation e.g. substitution, for improved binding to MHC claim I molecules e.g. a substitution of the amino acid residues at position 2 to leucine or methionine and/or substitution to valine at position 9 for HLA-A2,
ii) which comprises an amino acid substitution at position 2 and/or position 9 of the peptide,
iii) wherein the variant comprises an amino acid substitution which replaces the amino acid residue at position 2 with (1) a glycine residue; (2) leucine residue (3) methionine or (4) valine residue and/or
iv) wherein the variant comprises an additional group e.g. a methylene group at position 4.

4. A peptide according to claim 3, which has a contiguous amino acid sequence of at least 7 amino acid residues that are comprised as a contiguous sequence within the region of the HER2/neu protein to which the antibody binds.

5. A peptide according to any preceding claim, which is obtainable by a method comprising:
(a) binding an anti HER2/neu antibody to a Her/neu protein to form a complex;
(b) subjecting the complex to proteasome digestion;
(c) obtaining the digestion product comprising peptides; and
(d) identifying an immunogenic peptide sequence from the digestion products.

6. A peptide according to any preceding claim, which consists of between 7 to 30 amino acid residues, and optionally between 7 and 20 amino acid residues and further optionally between 7 and 15 amino acid residues, and further optionally 7 and 12 amino acid residues, and further optionally between 8 and 10 amino acid residues e.g. 9 amino acid residues.

7. A peptide according to claim 1, wherein the peptide has the amino acid sequence of SEQ ID NO: 4, 5, 6, 7, 8, 9, 10, 11, 12, 13, 15, 16, 17, or 18.

8. A peptide according to any preceding claim, for use with a cytokine, wherein optionally said cytokine is GM-CSF.

9. A therapeutically effective composition comprising a peptide that represents a portion of the sequence of HER2/neu antigen, wherein the peptide contains the sequence of SEQ ID NO: 4, and further comprising an antibody that binds to an immunogenic region of the antigen, wherein optionally the antibody is trastuzumab.

10. A vaccine comprising the composition of claim 9, which is therapeutically effective in the treatment of cancer, wherein optionally the cancer is breast cancer.

11. A pharmaceutical composition for use in the treatment of cancer comprising a peptide that represents a portion of the sequence of HER2/neu antigen, wherein the peptide contains the sequence of SEQ ID NO: 4, and further comprising an antibody that binds to an immunogenic region of the antigen, wherein the antibody is trastuzumab.

12. A peptide which represents a portion of the sequence of HER2/neu antigen, and contains the sequence of SEQ ID NO: 4 for treating a patient in a method comprising, in any order:
a. administering a therapeutically effective amount of an antibody to the patient, wherein the antibody is trastuzumab; and
b. administering a therapeutically effective amount of the peptide to the patient.

13. The peptide of claim 12, wherein the peptide is administered before, after or at the same time as administration of the antibody.

14. The peptide of claim 13, wherein administration of the peptide and administration of the antibody have a synergistic effect on the patient.


SEQUENCE LISTING

<110> HENRY M. JACKSON FOUNDATION FOR THE ADVANCEMENT OF
      MILITARY MEDICINE, INC.

<120> TARGETED IDENTIFICATION OF IMMUNOGENIC PEPTIDES

<130> 8124.014.PCT

<140> PCT/US06/035171
<141> 2006-09-08

<150> 60/714,865
<151> 2005-09-08

<160> 20

<170> PatentIn Ver. 3.3

<210> 1
<211> 1255
<212> PRT
<213> Artificial Sequence

<220>
<223> Description of Artificial Sequence: Synthetic
      construct

<400> 1


<210> 2
<211> 9
<212> PRT
<213> Artificial Sequence

<220>
<223> Description of Artificial Sequence: Synthetic
      peptide

<400> 2
Lys Ile Phe Gly Ser Leu Ala Phe Leu
1               5


<210> 3
<211> 53
<212> PRT
<213> Artificial Sequence

<220>
<223> Description of Artificial Sequence: Synthetic
      polypeptide

<400> 3
Pro Arg Glu Tyr Val Asn Ala Arg His Cys Leu Pro Cys His Pro Glu
1               5                   10                  15

Cys Gln Pro Gln Asn Gly Ser Val Thr Cys Phe Gly Pro Glu Ala Asp
            20                  25                  30

Gln Cys Val Ala Cys Ala His Tyr Lys Asp Pro Pro Phe Cys Val Ala
        35                  40                  45

Arg Cys Pro Ser Gly
    50


<210> 4
<211> 9
<212> PRT
<213> Artificial Sequence

<220>
<223> Description of Artificial Sequence: Synthetic
      peptide

<400> 4

Phe Gly Pro Glu Ala Asp Gln Cys Val
1               5

<210> 5
<211> 9
<212> PRT
<213> Artificial Sequence

<220>
<223> Description of Artificial Sequence: Synthetic
      peptide

<400> 5
Ser Val Thr Cys Phe Gly Pro Glu Ala
1               5

<210> 6
<211> 9
<212> PRT
<213> Artificial Sequence

<220>
<223> Description of Artificial Sequence: Synthetic
      peptide

<400> 6
Cys Gln Pro Gln Asn Gly Ser Val Thr
1               5

<210> 7
<211> 9
<212> PRT
<213> Artificial Sequence

<220>
<223> Description of Artificial Sequence: Synthetic
      peptide

<400> 7
Arg Glu Tyr Val Asn Ala Arg His Cys
1               5

<210> 8
<211> 9
<212> PRT
<213> Artificial Sequence

<220>
<223> Description of Artificial Sequence: Synthetic
      peptide

<400> 8
Ala His Tyr Lys Asp Pro Pro Phe Cys
1               5

<210> 9
<211> 9
<212> PRT

<213> Artificial Sequence

<220>
<223> Description of Artificial Sequence: Synthetic
      peptide

<400> 9
Val Asn Ala Arg His Cys Leu Pro Cys
1               5


<210> 10
<211> 9
<212> PRT
<213> Artificial Sequence

<220>
<223> Description of Artificial Sequence: Synthetic
      peptide

<400> 10
Gln Pro Gln Asn Gly Ser Val Thr Cys
1               5


<210> 11
<211> 9
<212> PRT
<213> Artificial Sequence

<220>
<223> Description of Artificial Sequence: Synthetic
      peptide

<400> 11
Tyr Lys Asp Pro Pro Phe Cys Val Ala
1               5


<210> 12
<211> 9
<212> PRT
<213> Artificial Sequence

<220>
<223> Description of Artificial Sequence: Synthetic
      peptide

<400> 12
Phe Leu Pro Glu Ala Asp Gln Cys Val
1               5


<210> 13
<211> 9
<212> PRT
<213> Artificial Sequence

<220>
<223> Description of Artificial Sequence: Synthetic
      peptide

<400> 13

Gly Ile Leu Gly Phe Val Phe Thr Leu
1               5

<210> 14
<211> 9
<212> PRT
<213> Artificial Sequence

<220>
<223> Description of Artificial Sequence: Synthetic
      peptide

<400> 14
Lys Ile Phe Gly Ser Leu Ala Phe Leu
1               5

<210> 15
<211> 9
<212> PRT
<213> Artificial Sequence

<220>
<223> Description of Artificial Sequence: Synthetic
      peptide

<400> 15
Ile Met Asp Gln Val Pro Phe Ser Val
1               5

<210> 16
<211> 9
<212> PRT
<213> Artificial Sequence

<220>
<223> Description of Artificial Sequence: Synthetic
      peptide

<400> 16
Arg Leu Leu Gln Glu Thr Glu Leu Val
1               5

<210> 17
<211> 9
<212> PRT
<213> Artificial Sequence

<220>
<223> Description of Artificial Sequence: Synthetic
      peptide

<400> 17
Phe Val Pro Glu Ala Asp Gln Cys Val
1               5

<210> 18
<211> 9
<212> PRT

<213> Artificial Sequence

<220>
<223> Description of Artificial Sequence: Synthetic
      peptide

<400> 18
Phe Gly Pro Glu Ala Asp Gln Cys Val
  1               5


<210> 19
<211> 9
<212> PRT
<213> Artificial Sequence

<220>
<223> Description of Artificial Sequence: Synthetic
      peptide

<400> 19
Ile Ile Ser Ala Val Val Gly Ile Leu
  1               5


<210> 20
<211> 9
<212> PRT
<213> Artificial Sequence

<220>
<223> Description of Artificial Sequence: Synthetic
      peptide

<400> 20
Ile Val Ser Ala Val Val Gly Ile Leu
  1               5